# DEMANDE DE BREVET EUROPEEN

(11) **EP 0 961 060 A1**
(43) Date de publication de la demande: **01.12.1999**
(21) Numéro de dépôt: 99401263.1
(22) Date de dépôt: 27.05.1999
(51) Int. Cl.: F16K 11/085

(54) **Robinet à boisseau et à clé tournante à voies de communication multiples**

(30) Priorité: 28.05.1998 FR 9806735
(71) Demandeur: VYGON, F-95440 Ecouen (FR)
(72) Inventeur: Brinon, Thierry, 95560 Montsoult (FR)
(74) Mandataire: Schrimpf, Robert

(57) **Abrégé**

Un robinet du type comportant une « clé » (30) pouvant tourner dans un logement cylindrique d'un corps ou « boisseau » (10), le boisseau présentant deux premiers percements (11, 13) diamétralement opposés, qui constituent une voie passante lorsqu'ils sont mis en communication par un percement traversant (31) de la clé dans une première position de fonctionnement de ladite clé, le boisseau comportant également un troisième conduit (12) qui est mis en communication avec ladite voie passante dans ladite première position de fonctionnement de la clé grâce à un deuxième percement (33) de la clé, caractérisé en ce que la clé comporte un troisième percement (32), traversant, qui, dans une deuxième position de fonctionnement de la clé, met en relation exclusivement les deux premiers conduits diamétralement opposés du boisseau (11, 13).

## Description

La présente invention concerne les robinets à clé tournante, notamment ceux utilisés dans le génie médical, en particulier pour constituer des rampes de robinets, par exemple pour réaliser des circuits de perfusion.

Ces robinets comportent un corps creux (dit boisseau), dont la paroi comporte des conduits permettant le raccordement de canalisations et dans lequel tourne un cylindre plein complémentaire du creux du boisseau (dit clé), pourvu de percements en sorte que la rotation de ladite clé commande la communication entre les conduits formés dans la paroi du boisseau.

L'invention concerne plus particulièrement les robinets dont le boisseau présente deux premiers conduits diamétralement opposés et un troisième conduit à angle droit desdits conduits opposés, la clé présentant un percement en T qui, dans une première position de la clé, fait communiquer les trois conduits du boisseau (robinets dits « à trois voies »).

Ces robinets présentent de nombreuses possibilités de raccordement entre les conduits du boisseau, selon la position de la clé, ce qui peut entraîner des erreurs de manipulation.

De fait, l'intérêt se présente de réaliser un robinet offrant seulement deux possibilités de raccordement correspondant à deux positions de fonctionnement de la clé, et telles que dans chacune de ces deux positions, les deux conduits opposés du boisseau communiquent au travers de la clé alors que dans une seule de ces deux positions, ces deux conduits communiquent également avec le troisième conduit du boisseau.

Un tel robinet fonctionnerait en quelque sorte comme un raccord en T sur la branche latérale duquel est monté un robinet une voie.

On y parvient selon l'invention, par le fait que la clé du robinet, outre le percement en T défini plus haut comporte un autre percement qui est rectiligne et traversant, et qui, dans une deuxième position de fonctionnement de la clé établit une communication exclusivement entre lesdits conduits diamétralement opposés du boisseau.

Des aspects préférés, mais non limitatifs, du robinet selon l'invention sont les suivants :
- le robinet comprend des moyens sur la clé et sur une partie fixe du robinet pour définir deux butées correspondant respectivement aux deux positions de fonctionnement du robinet et limitant la rotation de ladite clé entre ces deux positions de fonctionnement, les positions de fonctionnement de la clé étant limitées aux dites deux positions de fonctionnement correspondant respectivement aux deux butées,
- la clé est coiffée d'un index permettant de visualiser la position angulaire de la clé dans le boisseau,
- l'angle entre les deux positions de fonctionnement de la clé est de 45°,
- la clé ne comporte aucun percement sur une portion de circonférence de la clé se trouvant en regard d'un des conduits du boisseau lors de la rotation de la clé entre ses deux positions de fonctionnement,
- il est prévu des moyens pour faire basculer la clé vers une des première ou deuxième positions de fonctionnement lorsque ladite clé se trouve entre ces deux positions, et pour maintenir la clé dans ladite première ou deuxième position de fonctionnement.

En outre, l'invention propose également une rampe réalisée par la succession de robinets selon l'une quelconque des caractéristiques énoncées ci-dessus et dont les voies passantes sont alignées, ainsi qu'une rampe réalisée en montant en série des robinets à une, deux ou trois voies et des robinets réalisés selon l'une quelconque des caractéristiques énoncées ci-dessus.

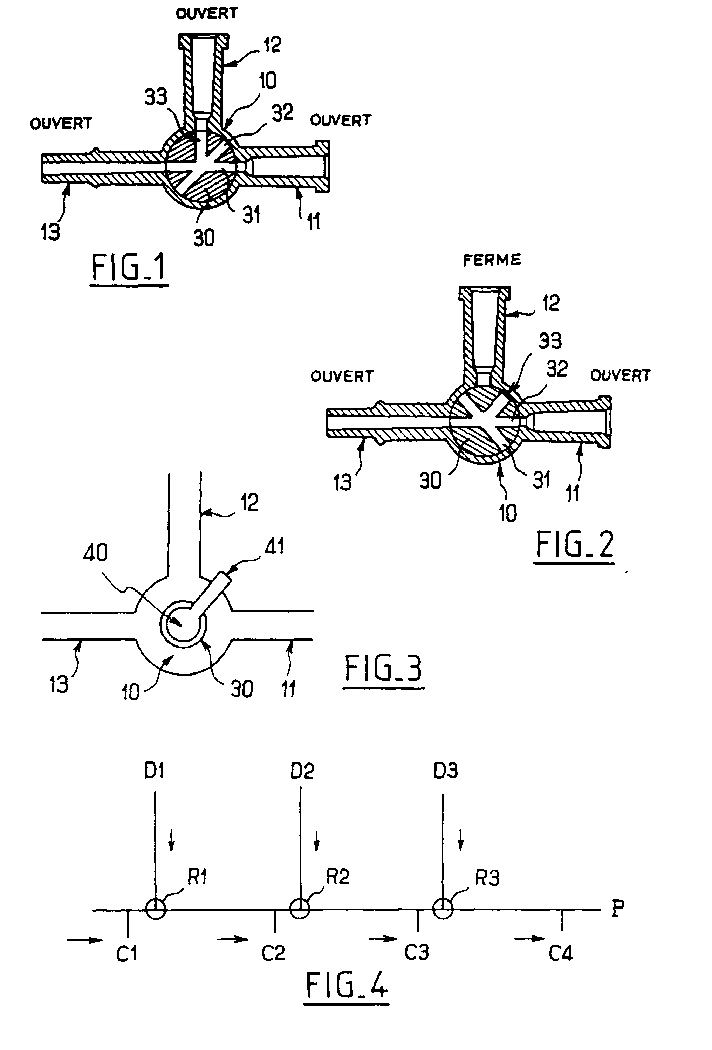

D'autres aspects, buts et avantages de la présente invention apparaîtront mieux à la lecture de la description détaillée suivante d'une forme de réalisation préférée de celle-ci, donnée à titre d'exemple et faite en référence aux dessins annexés, sur lesquels :
. la figure 1 est une représentation schématique en coupe horizontale d'un robinet réalisé selon l'invention en position « voie latérale ouverte »,
. la figure 2 est une représentation schématique en coupe horizontale d'un robinet réalisé selon l'invention en position « voie latérale fermée »,
. la figure 3 est une représentation schématique en vue de dessus d'un robinet réalisé selon l'invention,

- la figure 4 est une représentation schématique d'un rampe de robinets utilisant des robinets selon l'invention.

Les figures 1 et 2 sont des représentations similaires du robinet dans ses deux positions de fonctionnement possibles. Les trois conduits 11, 12 et 13, munis de conduits de raccordement respectifs relient le boisseau 10 à des canalisations externes, non représentées sur les figures.

La clé 30 est munie de trois percements 31, 32 et 33 se rejoignant en son centre. Ces trois percements s'étendent dans le même plan orthogonal à l'axe de rotation de la clé dans le boisseau; ils sont également coplanaires aux axes des conduits 11, 12 et 13 lorsque la clé est montée dans le boisseau.

La position « voie latérale ouverte » du robinet est représentée schématiquement sur la figure 1 : les trois conduits sont en communication avec les percements 31, 32 et 33 et laissent donc passer le fluide dans la voie latérale de dérivation raccordée au conduit 12, la voie principale joignant les conduits 11 et 13 étant également passante.

Sur la figure 2, seule la voie principale est passante grâce à la communication des deux conduits 11 et 13 par l'intermédiaire du percement 32, le troisième conduit 12 ne laissant pas passer le fluide; le robinet est alors en position « voie latérale fermée ».

Ainsi apparaît-il qu'un robinet réalisé selon l'invention commande l'arrivée de fluide par une voie de dérivation en laissant, dans chacune de ses deux positions de fonctionnement possibles, passer le fluide dans une voie principale.

Une vue externe du robinet est représentée sur la figure 3. On y voit un index 41, coiffant la clé et solidaire de celle-ci dans sa rotation. Le rôle de cet index est d'indiquer clairement à l'utilisateur si le robinet est en position « voie latérale ouverte » ou « voie latérale fermée ». Lorsque le robinet est en position « voie latérale ouverte », l'index est situé à l'aplomb du percement 33, et se trouve en regard du conduit 12. Lorsque le robinet est en position « voie latérale fermée », l'index est décalé de 45° et ne se trouve en face d'aucun des conduits 11, 12, 13. On permet ainsi à l'utilisateur de visualiser instantanément et sans ambiguïté la position dans laquelle se trouve le robinet. Cette disposition est particulièrement importante dans un contexte sanitaire ou hospitalier, qui exige une grande sûreté de fonctionnement des installations.

Selon une première caractéristique non représentée sur les figures, le robinet selon l'invention comprend des moyens de limitation de la rotation de la clé dans le boisseau dans une plage angulaire pouvant être de 45° par exemple. De tels moyens de limitation de rotation, qui peuvent par exemple utiliser la coopération entre une partie saillante de la clé mobile et une cavité d'une partie fixe solidaire du boisseau, ou inversement, sont connus de l'homme du métier et ne seront donc pas décrits plus en détail dans le présent texte.

Selon une deuxième caractéristique non représentée sur les figures, le robinet selon l'invention peut également comprendre des moyens pour maintenir la clé dans une de ses deux positions de fonctionnement en l'absence de toute intervention d'un opérateur.

Ces moyens peuvent consister en des parties en creux et des protubérances portées respectivement par la clé et une partie fixe solidaire du boisseau du robinet, ou inversement, la coopération entre ces parties assurant en outre que la clé du robinet ne peut être laissée accidentellement dans une position intermédiaire entre les positions « voie latérale ouverte » et « voie latérale fermée ».

Selon cette deuxième caractéristique, les parties en creux et les protubérances sont dimensionnées de manière à se déformer élastiquement pour permettre à un opérateur de faire passer par forçage la clé d'une position de fonctionnement du robinet à l'autre.

Le robinet selon l'invention peut être utilisé isolément, ou dans des rampes qui intègrent plusieurs robinets. De telles rampes sont couramment utilisées en génie médical, par exemple dans les circuits de perfusion. La figure 4 est une représentation schématique d'une rampe constituée de trois robinets R1, R2 et R3 selon l'invention.

Les conduits diamétralement opposés de ces robinets sont reliés à des canalisations alignées C1, C2, C3 et C4 formant une voie principale P, alors que le troisième conduit de chaque robinet est relié à une canalisation de dérivation D1 à D3. Quelle que soit la position de fonctionnement des robinets, la voie P est passante. Par contre, les robinets R1, R2 et R3 commandent le raccordement des dérivations D1, D2 et D3 respectivement à la canalisation principale P.

Selon un autre mode de réalisation d'une rampe comportant des robinets selon l'invention, il est également possible d'intégrer dans une rampe des robinets à une voie, deux voies ou trois voies connus par ailleurs en combinaison avec des robinets selon l'invention, pour multiplier les possibilités de raccordement de canalisation.

Il apparaît donc que le robinet selon l'invention met en oeuvre un boisseau trois voies de type connu. Il est ainsi possible d'utiliser de tels boisseaux avec une clé trois voies classique pour constituer un robinet à trois voies, ou avec une clé telle que décrite ci-dessus pour constituer un robinet selon l'invention.

Cette caractéristique est avantageuse pour les fabricants, qui peuvent proposer des robinets à trois voies, des robinets selon l'invention et des rampes mettant en oeuvre les deux types de robinet, en investissant seulement dans les moules nécessaires à la fabrication d'un type de boisseau unique. De plus, les stocks de semi ouvrés correspondants (boisseaux de robinets ou corps de rampes) peuvent être limités à un seul type, ce qui réduit l'en cours financier et simplifie la gestion des stocks. Le montage final d'un des deux types de clé (standard ou selon l'invention) dans le boisseau de type unique suffit alors à spécialiser le robinet ou la rampe.

Dans le cas particulier des rampes, cette caractéristique permet de réaliser très simplement une gamme très large de configurations en jouant sur la combinaison des deux types de clés.

Bien entendu, la présente invention n'est nullement limitée au mode de réalisation présenté, mais l'homme du métier saura y apporter toutes variantes ou modifications conformes à son esprit.

## Revendications

1. Robinet du type comportant une clé (30) pouvant tourner dans un logement cylindrique d'un corps ou « boisseau » (10), le boisseau présentant deux premiers conduits (11, 13) diamétralement opposés, qui constituent une voie passante lorsqu'ils sont mis en communication par un percement traversant (31) de la clé dans une première position de fonctionnement de ladite clé, le boisseau comportant également un troisième conduit (12) qui est mis en communication avec ladite voie passante dans ladite première position de fonctionnement de la clé grâce à un deuxième percement (33) de la clé, caractérisé en ce que la clé comporte un troisième percement (32), traversant, qui, dans une deuxième position de fonctionnement de la clé, met en relation exclusivement les deux premiers conduits diamétralement opposés du boisseau (11, 13).

2. Robinet selon la revendication 1, caractérisé en ce qu'il comprend des moyens sur la clé (30) et sur une partie fixe du robinet pour définir deux butées correspondant respectivement aux deux positions de fonctionnement du robinet et limitant la rotation de ladite clé entre ces deux positions de fonctionnement, les positions de fonctionnement de la clé étant limitées aux dites deux positions de fonctionnement correspondant respectivement aux deux butées.

3. Robinet selon la revendication 1 ou 2, caractérisé en ce que la clé (30) est coiffée d'un index (41) permettant de visualiser la position angulaire de la clé dans le boisseau.

4. Robinet selon l'une quelconque des revendications 1 à 3, caractérisé en ce que l'angle entre les deux positions de fonctionnement de la clé est de 45°.

5. Robinet selon l'une quelconque des revendications 1 à 4, caractérisé en ce que la clé ne comporte aucun percement sur une portion de circonférence de la clé se trouvant en regard d'un des conduits du boisseau lors de la rotation de la clé entre ses deux positions de fonctionnement.

6. Robinet selon l'une quelconque des revendications 1 à 5, caractérisé en ce qu'il est prévu des moyens pour faire basculer la clé (30) vers une des première ou deuxième positions de fonctionnement lorsque ladite clé se trouve entre ces deux positions, et pour maintenir la clé dans ladite première ou deuxième position de fonctionnement.

7. Rampe réalisée par la succession de robinets selon l'une quelconque des revendications 1 à 6, dont les voies passantes sont alignées.

8. Rampe réalisée en montant en série des robinets à une, deux ou trois voies et des robinets réalisés selon l'une quelconque des revendications 1 à 6.
